# EUROPEAN PATENT APPLICATION

(11) **EP 1 588 719 A1**
(43) Date of publication of application: **26.10.2005**
(21) Application number: 05008173.6
(22) Date of filing: 14.04.2005
(51) Int. Cl.: A61L 2/02, A61L 2/04, A61L 2/26

(54) **Sterile closure apparatus**

(30) Priority: 16.04.2004 JP 2004121692
(71) Applicant: Shibuya Machinery Co., Ltd., Kanazawa-shi, Ishikawa (JP)
(72) Inventor: Nishimura, Noboru, Kanazawa-hi Ishikawa (JP)
(74) Representative: HOFFMANN EITLE

(57) **Abstract**

A sterile closure apparatus 1 is provided which seals an opening 6a formed in a partition wall 6 which partitions between a plurality of sterile spaces 2, 4. The apparatus comprises a shutter 8 of a size greater than the opening 6a, an air cylinder 20 for moving the shutter 8, and an annular seal member 22 mounted on the shutter and adapted to be brought into close contact with the wall surface around the opening 6a when it is inflated. The seal member 22 is mounted in a channel-shaped mounting groove 8b formed in the shutter 8, and is inflated when air is introduced into it. Fisrt and third heaters 24 and 28 are mounted on the wall surface of the partition wall 6 around the opening 6a against which the annular seal member 22 is urged into close contact and in the inner surface of the mounting groove 8b. During a sterilizing operation, the opening 6a is covered by the shutter 8, and the seal member 22 is inflated to be brought into close contact with the first heater 24 on the partition wall 6. Regions with which the seal member is brought into close contact are sterilized by the first and third heaters 24 and 28. The invention simplifies the construction of the sterile closure apparatus 1, allowing a sterilization step to be completed in a reduced length of time.

## Description

### BACKGROUND OF THE INVENTION AND RELATED ART STATEMENT

The present invention relates to a sterile closure apparatus formed on a partition wall which partitions between a plurality of spaces which are used in a sterile condition for closing an opening formed in the partition wall by a shutter.

A sterile treatment system is known in the art which includes a dry sterilizer and an isolator disposed adjacent to each other, with an opening formed in a partition wall which partitions between both spaces so that an article such as a vessel which is sterilized by the dry sterilizer can be carried into the isolator through the opening to be subject to a following treatment.

The opening formed in the partition wall which partitions two spaces in the manner mentioned above is constructed to be capable of being opened and closed by a shutter. When the interior of each space is to be sterilized, a gasket (seal member) attached to the shutter is urged against the periphery of the opening in the partition wall for purpose of sealing. However, a portion of the gasket which is in contact with the partition wall is maintained in close contact with the wall surface of the partition wall irrespective of the side on which the space to be sterilized is located , and accordingly, is kept out of contact with a sterilizer agent such as a sterilizing gas. In addition, a hot air from the dry sterilizer is hardly accessible to the gasket, which cannot be heated sufficiently, leaving a portion which remains unsterilized at any time.

Accordingly, the present Applicant has filed an application for an invention of a sterilizer capable of sterilizing the entirety inclusive of a gasket which is mounted on the wall surface around an opening formed in a partition wall and a shutter which is brought into close contact with the gasket (see Japanese Laid-Open Patent Publication No. 2002-65820).

A sterilizer disclosed in this citation is adapted to be used with an isolator of a large volume, which is partitioned into a plurality of spaces by a partition wall having an opening formed therein, the opening being opened or closed by a shutter and seal members. Specifically, a first annular seal member is disposed around the opening in the partition wall, and a second annular seal member is disposed around the outer periphery of the first seal member. These seal members can be inflated by introducing compressed air therein to be in close contact with the shutter.

In the sterilizer mentioned above, when one of the spaces which are partitioned by the partition wall is to be sterilized, one of the seal members is inflated to be in close contact with the shutter, while the space in which the seal member which is not inflated is exposed is sterilized. When the other space is to be sterilized, the other seal member is inflated, while the space in which the seal member which is not inflated is exposed is sterilized. When the spaces on the opposite sides are successively sterilized, the two seal members are also sterilized.

The sterilizer disclosed in the citation requires a double disposition of annular seal members and the sterilization must be performed in two steps, presenting problems of a complex construction, an increased cost and an increased length of working time.

### OBJECT AND SUMMARY OF THE INVENTION

Accordingly, it is an object of the present invention to provide a sterile closure apparatus which is simple in construction and which can be manufactured inexpensively and capable of drastically reducing a length of time required for a sterilizing operation.

Above object is accomplished by providing a sterile closure apparatus comprising a shutter having a greater area than an opening formed in a partition wall which partitions between a plurality of sterile spaces, moving means for moving the shutter to a position where it covers the opening and a position where it opens the opening, an annular seal member disposed on either the shutter or around the opening in the partition wall and adapted to be inflated to seal between the shutter and the partition wall to close the opening, and a heater for heating the surface of the shutter or a wall surface around the opening against which the annular seal member is in close contact when it is inflated, thus sterilizing the annular seal member which is in close contact therewith.

With the sterile closure apparatus according to the present invention, during a normal operation in which articles such as vessels are treated, the shutter is moved so as to open the opening in the partition wall. On the other hand, when each space is to be sterilized, the shutter is moved to a position where it covers the opening, and the annular seal member is inflated to be brought into close contact with the mating member to seal between the shutter and the partition wall. In a conventional arrangement, it has been impossible to sterilize a portion of the seal member which is in close contact with the mating shutter or partition wall, but according to the present invention, a heater is provided on either a shutter or a wall surface around the opening with which the seal member is in close contact, to heat the surface to sterilize the seal member which is in contact therewith.

### BRIEF DESCRIPTION OF THE DRAWINGS

Fig. 1 is a longitudinal section of a sterile closure apparatus according to one embodiment of the present invention which maintains a sterile space partitioned by a partition wall having an opening formed therein in a sealed condition;
Fig. 2 is a front view showing the opening and the shutter;
Fig. 3 is a cross section to an enlarged scale of an essential part of Fig. 1; and
Fig. 4 is a cross section to an enlarged scale of an essential part of a sterile closure apparatus according to another embodiment.

### DETAILED DESCRIPTION OF PREFERRED EMBODIMENTS

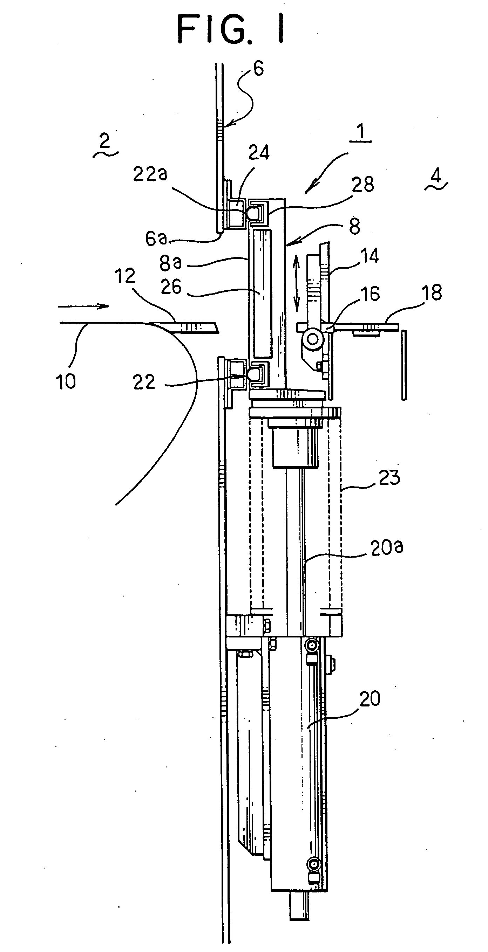

Referring to the drawings, several embodiments of the invention will be described below. In Fig. 1, there are shown a dry sterilizer 2 which is used for a dry sterilization of vials at an upstream position or to the left as viewed in this Figure, and an isolator 4 in which a filling equipment, not shown, at a downstream position, and spaces 2 and 4 are partitioned by a partition wall 6.

The partition wall 6 which partitions between the dry sterilizer 2 and the isolator 4 is formed with an opening 6a which provides a communication between the both spaces 2 and 4 for a hand-over of vessels such as vials. A conveyer 10 which conveys vessels continuously is disposed in the dry sterilizer 2, and vessels are subject to a dry sterilization during the time they are conveyed on the conveyer 10, and are driven into the opening 6a through a stationary transition plate 12 which is disposed at the downstream end of the conveyer 10. These vessels are transferred to a movable transition plate 14 which assumes a horizontal position during a normal operation and which is rotatable within the isolator 4 and are then handed over to a conveyer 18 disposed within the isolator 2 through a stationary transition plate 16 to be conveyed to the filling equipment mentioned above. The movable transition plate 14 is rotatable between a horizontal position and an upright position shown in Fig. 1. During a normal operation, the transition plate 14 assumes a horizontal position to bridge between the transition plate 12 in the dry sterilizer 2 and the stationary transition plate 16 in the isolator 4. When the opening 6a is closed by a shutter 8 as will be described below, the transition plate 14 assumes its upright position shown in Fig. 1 to avoid an interference with the shutter 8.

Both the dry sterilizer 2 and the isolator 4 need to have their interior sterilized. A sterile closure apparatus 1 according to one embodiment of the present invention is provided to permit the opening 6a through which the vessels are handed over to be tightly closed during a sterilizing operation. The sterile closure apparatus 1 comprises an air cylinder 20 which is secured to a surface of the partition wall 6 which faces the isolator 4 in a upwardly oriented vertical position and acting as means for moving the shutter 8, the shutter 8 secured to the piston rod 20a of the air cylinder 20 and elevatable between a position where it opposes the opening 6a in the partition wall 6 and a downward position located below the opening 6a, and an annular seal member 22 mounted on the shutter 8. The shutter 8 has a greater area than the opening 6a so that when it is moved to the position opposing the opening 6a in the partition wall 6, it can cover the entire opening 6a. Bellows 23 are connected between the top end of the air cylinder 20 and the free end of the piston rod 20a for covering a sliding portion of the air cylinder 20.

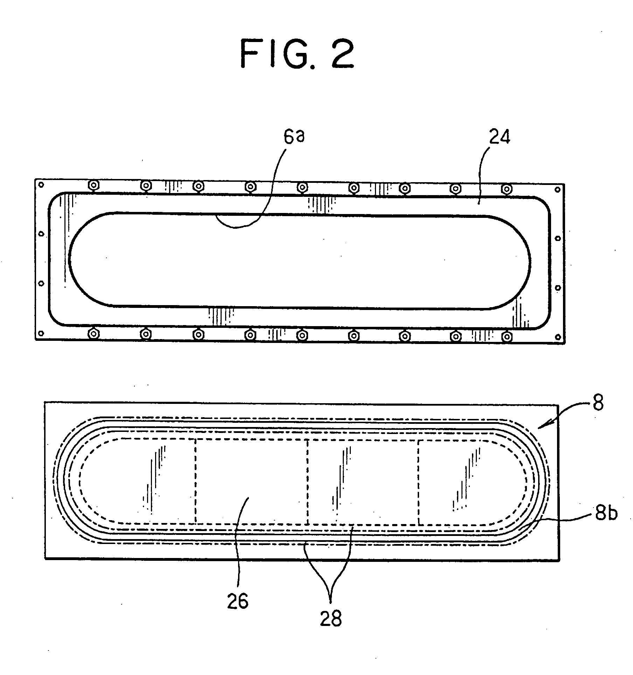

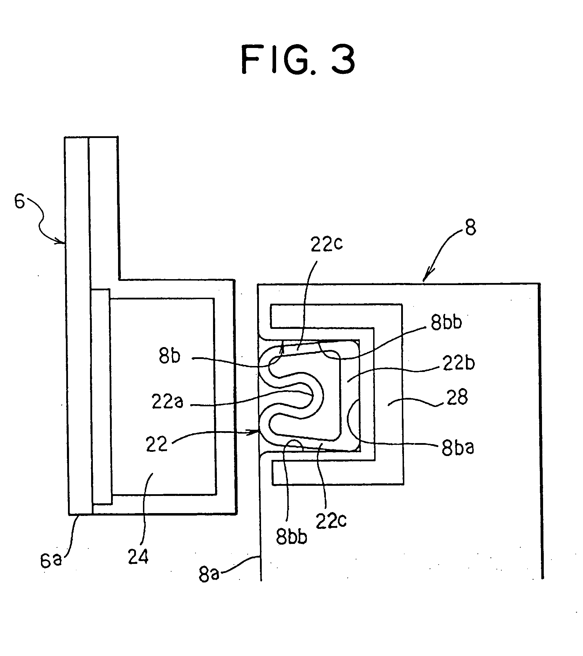

Referring to Figs. 2 and 3, structures of the opening 6a in the partition wall 6 and the shutter 8 which seals the opening 6a will be described. The opening 6a is horizontally elongate and have arcuate opposite ends, and a first heater 24 is mounted on the wall surface of the partition wall 6 which surrounds the opening 6a. The first heater 24 comprises an electric heating wire.

On the other hand, a second heater 26 (refer Fig. 1) of a size commensurate with the area of the opening 6a is embedded in the surface 8a of the shutter 8 which faces the opening 6a. A groove (seal member receiving groove) 8b which is channel-shaped in section and substantially conforming to the configuration of the first heater 24 around the opening 6a is formed toward the outer periphery of the shutter 8. The open side of the channel-shaped groove 8b faces the partition wall 6, and the annular seal member 22 is mounted in the groove 8b so as to be located opposite to the first heater 24 around the opening 6a. The annular seal member 22 has a bottom surface 22b which is adhesively bonded to the bottom surface 8ba of the channel-shaped groove 8b, but no other part is secured, allowing the seal member to be freely deformed.

The annular seal member 22 is formed of a material such as silicone rubber, for example, which is expandable and shrinkable, and its interior is connected to a source of air supply, not shown. It can be inflated by introducing compressed air into the annular seal member 22 from the source of air supply. The annular seal member 22 shown in Fig. 1 indicates an inflated condition when compressed air is introduced into the interior of the seal member. In its inflated condition, its front end face 22a projects out of the seal member mounting groove 8b in the shutter 8 and is in close contact with the surface of the first heater 24 which is mounted around the opening 6a in the partition wall 6. At this time, both lateral portions 22c, 22c of the annular seal member 22 are in close contact with opposite sidewalls 8bb, 8bb of the channel-shaped groove 8b. The annular seal member 22 shown in Fig. 3 indicates a condition in which the air is exhausted from the interior, and the front and 22a is retracted and spaced from the first heater 24 around the opening 6a, and is received within the seal member mounting groove 8b. It will be seen that the both lateral portions 22c, 22c are also spaced from the opposite sidewalls 8bb, 8bb of the channel-shaped groove 8b.

A third heater 28 comprising an electric heating wire is embedded at a location within the shutter 8 which correspond to the bottom 8ba and both sidewalls 8bb, 8bb of the channel-shaped groove 8b in which the annular seal member 22 is received.

An operation of the sterile closure apparatus 1 constructed in the manner mentioned above will now be described. When vessels such as vials are to be filled under a sterile condition, the air cylinder 20 is actuated to lower the shutter 8 to open the opening 6a formed in the partition wall 6 which partitions between the dry sterilizer 2 and the isolator 4, and the movable transition plate 14 which is disposed within the isolator 4 is thrown to its horizontal position so as to bridge between the stationary transition plate 12 within the dry sterilizer 2 and the stationary transition plate 16 within the isolator 4.

Under this condition, vessels are introduced into the dry sterilizer 2 to perform a drying sterilization while the vessels are continuously conveyed by the conveyer 10. The vessels which have been subject to the drying sterilization are transferred successively onto the transition plate 12 as urged by the succeeding vessels on the conveyer 10, and are transferred onto the movable transition plate 14 within the isolator 4 to pass through the opening 6a in the partition wall 6 to be transferred onto the stationary transition plate 16, whereupon they are placed on the conveyer 18 within the isolator 4 to be fed to a filling equipment, not shown, for purpose of a filling operation.

When performing a sterilization of the dry sterilizer 2 and the isolator 4, the movable transition plate 14 within the isolator 4 is initially flipped to its upright position, and the air cylinder 20 is actuated to raise the shutter 8 until it is located opposite to the opening 6a in the partition wall 6. As the compressed air from the source of air supply is introduced into the annular seal member 22, the seal member becomes inflated, with its front end surface 22a projecting out of the groove 8b and urged against the surface of the first heater 24 which is disposed on the wall surface around the opening 6a. The both lateral portions 22c, 22c of the annular seal member 22 are brought into close contact with the opposite sidewalls 8bb, 8bb of the channel-shaped groove 8b.

After the opening 6a in the partition 6 disposed between the dry sterilizer 2 and the isolator 4 are sealed by the shutter 8 and the annular seal member 22 which is mounted on the shutter 8, both the dry sterilizer 2 and the isolator 4 are subject to the sterilizing operation. The interior of the dry sterilizer 2 is sterilized by blowing a hot air, and the interior of the isolator 4 is sterilized by vapor of hydrogen peroxide. The surface 8a of the shutter 8 which covers the opening 6a is exposed within the dry sterilizer 2, but the hot air is hardly accessible to the surface 8a. Accordingly, the second heater 26 embedded below the surface 8a of the shutter 8 is used to sterilize the surface 8a.

Since the front end surface 22a of the annular seal member 22 is in close contact with the surface of the first heater 24 which is mounted on the wall surface around the opening 6a and the both lateral portions 22c, 22c of the annular seal member 22 are in close contact with the opposite sidewalls 8bb, 8bb of the channel-shaped groove 8b to prevent a contact with the hot air from the dry sterilizer 2 and vapor of hydrogen peroxide within the isolator 4, thus preventing a sterilization by the heated air or vapor of hydrogen peroxide. However, they are in contact with the first and third heaters 24 and 28, the heat of which is effective to achieve a sterilization.

In this manner, if the single annular seal member 22 is used to seal, this annular seal member 22 can be sterilized, and thus it is possible to sterilize the entirety including the interior of the dry sterilizer 2 and the isolator 4 with a simple arrangement as compared with the prior art and in a simple step. While the embodiment has been described in which the partition wall 6 is used to partition between the dry sterilizer 2 and the isolator 4, the invention is not limited in its application thereto, but is equally applicable to an arrangement in which a large sized isolator is partitioned into a plurality of chambers as disclosed in the citation.

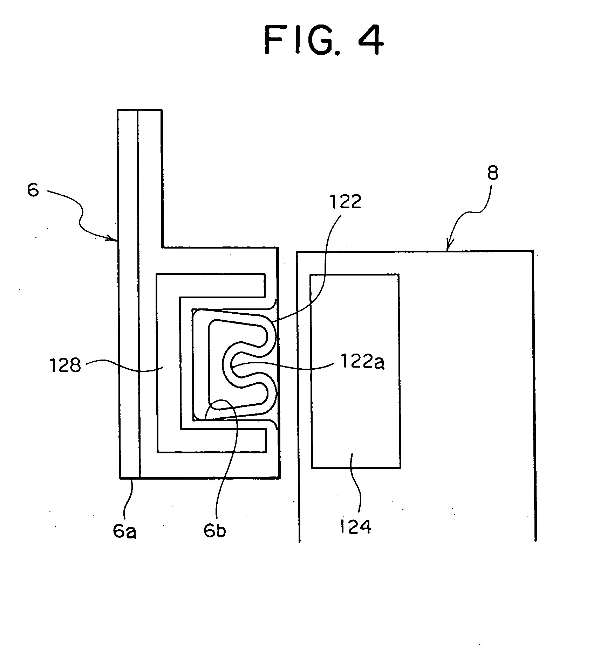

While the annular seal member 22 is mounted on the shutter 8 in the described embodiment, it is also possible to dispose the annular seal member on the wall surface of the partition wall 6 around the opening 6a. In this instance, as indicated in Fig. 4, the partition wall 6 is formed with a mounting groove 6b which receives an annular seal member 122 while the shutter 8 is provided with a heater 124 at a location which is in close contact with a front end 122a of the annular seal member 122, with a channel-shaped heater 128 being disposed on the partition wall 6 in surrounding relationship with the mounting groove 6b.

## Claims

1. A sterile closure apparatus comprising a shutter which is a greater area than an opening formed in a partition wall which partitions between a plurality of sterile spaces, moving means for moving the shutter to a position where it covers the opening and a position where it opens the opening, and an annular seal member disposed on either the shutter or on the partition wall around the opening for sealing between the shutter and the partition wall when it is inflated to close the opening;
**characterized by** a heater for heating either the wall surface around the opening or the surface of the shutter with which the annular seal member is in close contact when it is inflated, thereby sterilizing the annular seal member which is in close contact therewith.

2. A sterile closure apparatus according to Claim 1 in which an annular seal mounting groove is formed in either the shutter or around the opening for receiving the annular seal member therein.

3. A sterile closure apparatus according to Claim 2 in which a heater is disposed in the seal member mounting groove to heat the inner surface thereof to sterilize the annular seal member.

4. A sterile closure apparatus according to Claim 2 in which the annular seal member is formed of a material which is expandable and shrinkable, the interior of the annular seal member being connected to a source of air supply to make the annular seal member expandable and shrinkable by introducing air into the interior or exhausting air from the interior.

5. A sterile closure apparatus according to Claim 1 in which a heater of a size which is substantially commensurate with the area of the opening is provided in the surface of the shutter which faces the opening.
